(19) Europäisches Patentamt
European Patent Office
Office européen des brevets

(11) EP 2 340 072 B1

(12) **EUROPEAN PATENT SPECIFICATION**

(45) Date of publication and mention of the grant of the patent:
**02.06.2021 Bulletin 2021/22**

(21) Application number: **09740918.9**

(22) Date of filing: **28.09.2009**

(51) Int Cl.:
***A61M 15/08*** *(2006.01)*

(86) International application number:
**PCT/GB2009/002288**

(87) International publication number:
**WO 2010/040979 (15.04.2010 Gazette 2010/15)**

(54) **A METHOD FOR ADMINISTERING LIQUID ANALGESICS**
VERFAHREN ZUR VERABREICHUNG VON ANALGETIKA
MÉTHODE POUR ADMINISTRER DES ANALGÉSIQUES LIQUIDES

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **08.10.2008 GB 0818396**

(43) Date of publication of application:
**06.07.2011 Bulletin 2011/27**

(73) Proprietor: **Archimedes Development Limited**
**Nottingham NG2 2TN (GB)**

(72) Inventors:
- **CASTILE, Jonathan**
**Nottingham NG7 2TN (GB)**
- **WATTS, Peter**
**Nottingham NG7 2TN (GB)**
- **SMITH, Alan**
**Nottingham NG7 2TN (GB)**

(74) Representative: **HGF**
**1 City Walk**
**Leeds LS11 9DX (GB)**

(56) References cited:
**WO-A2-02/13886**
**US-A- 5 228 586**
**US-A1- 2004 135 002**
**US-A1- 2007 209 660**
**US-B1- 6 189 739**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

The present invention relates to a method for administering, in particular but not exclusively, opioid analgesics for the relief and treatment of pain.

The invention is particularly concerned with administration in the form of a spray such as in the intranasal, buccal or sublingual administration of drugs, or more particularly the intranasal administration of opioid analgesics.

Opioid analgesics are currently the most pharmacologically effective means of alleviating severe pain. Examples include morphine, diamorphine, codeine, hydromorphone, oxymorphone, oxycodone, meperidine, fentanyl, sufentanil, remifentanil and alfentanil.

Intranasal administration of opioid analgesics is especially advantageous since it provides the potential for very rapid treatment of acute pain since the drug molecule will rapidly pass from the nasal passages into the systemic circulation.

Although such rapid treatment is advantageous when the analgesic is used properly, it can be extremely hazardous to the health of a patient if the analgesic is accidentally or deliberately misused. For example misuse could lead to life threatening side effects, in particular respiratory depression.

As a consequence there are many legal and regulatory controls over the distribution, prescription and use of opioid analgesics.

For many therapeutic applications it is necessary to administer opioid analgesics several times throughout a day.

Bearing in mind the consequences of administering the opioid analgesic incorrectly, it will be appreciated that it is highly desirable to provide for the administration of several predetermined doses of an opioid analgesic in a manner which can be done simply and safely by a patient or carer; in particular in a manner which minimises the risk of administering too great a dose at any one time.

It is a general object of the present invention to provide a method for administration of an opioid analgesic in accordance with the aims set out above.

According to one aspect of the present invention there is provided a method of filling a device for administering a predefined number (N) of predefined volume unit doses (Vu) of an opioid analgesic solution, the device including a closed container containing a predetermined fill volume (Vf) of the opioid analgesic solution and a dispenser connected to the container, the dispenser being operable to administer an individual one volume unit dose (Vu) repeatedly said predefined number of times (N), both the dispenser being adapted and the concentration of said opioid analgesic in said solution being chosen such that said volume unit dose is in the range 0.05 to 0.15ml.

Preferably the volume unit dose is 0.1ml.

Typically, the concentration of the opioid analgesic in liquid form is in the range of 0.005 to 1000 mg/ml.

Optionally the opioid analgesic is a solution of fentanyl, the concentration of the fentanyl in the solution being in the range 0.1 to 20 mg/ml.

Preferably the opioid drug solution is a solution of fentanyl citrate, the concentration of fentanyl citrate in the solution being in the range of about 0.16 to 31.4 mg/ml.

The choice of the opioid solution as defined above which is dispensed in a volume unit dose in the range 0.05 to 0.15ml enables the device to be used to administer say one or maybe two volume unit doses at any one time to provide effective treatment. This means that at any one treatment time, the patient will be administered with either one volume unit dose into one nostril, or one volume unit dose into both nostrils. This makes it easy for the patient or carer who is administering the drug from the device to understand and remember the number of volume unit doses administered at one treatment time. This makes it much more unlikely that more than the prescribed number of doses will be administered and so makes it much more likely that the drug will be administered safely at any one treatment time.

It is recognised that there is a 'tailing-off' problem associated with administration of solutions from a nasal pump dispensing device viz. as the container empties full priming of the pump becomes less reliable; this in turn means that on actuation of the pump the volume of the drug solution dispensed can be less than the volume unit dose of the solution. Typically this means that there is a tendency for a patient or carer to administer an additional dose on realising that a full dose was not initially administered.

This can be a dangerous practice with analgesics of the potency of opioids.

Preferably, the dispenser includes counting means operable to count the number of one volume unit doses administered and further includes dispenser deactivating means which is operated by the counting means to prevent further administration of the drug by the dispenser when the counting means has counted said predefined number of times (N). This number (N) is chosen (bearing in mind the fill volume (Vf) of the solution contained in the container before any doses have been administered) in order to ensure that (N) full doses are administered, i.e. the deactivation means operates to prevent further discharge of solution from the device before the tail off phenomenon is reached.

This contributes to the safe use of the device as it gives to the patient or carer an indication as to the number of doses which have been administered and furthermore prevents administration of any solution remaining in the container after all the predefined number (N) of volume unit doses have been dispensed.

Preferably the predefined number (N) of volume unit doses is chosen to be in the range of 2 to 30, more preferably in the range 3-20, and most preferably the predefined number (N) is in the range 4 to 8.

Various aspects of the present invention are hereinafter described with reference to the accompanying drawings, in which:

Figure 1 is a schematic side view of a device according to a first embodiment of the present invention;
Figures 2A and 2B are, respectively, schematic side views of devices according to the first and second embodiments shown in a tipped condition;
Figures 3A, 3B, and 3C are, respectively, schematic side views of devices according to second, third and forth embodiments of the present invention; and
Figure 4 is a schematic side view of a device according to a fifth embodiment of the invention.

Referring initially to Figure 1 there is shown a nasal dispensing device 10 which includes a closed container 12 and a dispenser 14. An analgesic solution 15 is contained within the container 12.

The dispenser 14 is preferably a dispenser of the type disclosed in US patent 4565302. Other alternatives are disclosed in WO02/13886A2, US2004/135002A1, US2007/209660A1, US6189739B1, or US5228586A.

The dispenser 14 thereby includes a spray head 16 which is reciprocally mounted on a mounting body 18. The mounting body 18 is fixedly secured to the container 12 to prevent its removal; accordingly access to the solution 15 is not permitted unless it is dispensed via the dispenser 14. The body 18 may be attached to the container 12 by a crimp and snap fitting or a one-way screw fitting in which a ratchet on the body 18 engages with lugs on the container such that the body 18 can only be screwed in one direction only. An example of such a container can be purchased from Saint Gobain Desjonqueres, France.

The dispenser 14 further includes a pump 20 mounted on the mounting body 18. The pump 20 has an inlet (not shown) communicating with a dip tube 22. The dip tube 22 depends from the pump 20 towards the bottom of the container 12 such that its terminal end 23 is located beneath the surface of the solution 15. Accordingly on operation of the pump 20, solution is drawn along the tube 22 into the inlet of the pump 20.

The pump 20 further includes an outlet (not shown) which communicates with a spray nozzle 26 formed on the spray head 16. Accordingly on operation of the pump 20 solution is discharged through the spray nozzle 26 to form a spray 30.

The pump 20 is preferably of the two stroke reciprocating type having a priming chamber in which a pump piston reciprocates; the reciprocal motion of the piston in one direction, i.e. a first stroke of the piston, causing solution contained in the priming chamber to be discharged through the spray nozzle 26 and reciprocal motion of the piston in the opposite direction, i.e. a second stroke of the piston, causing solution to be drawn from the container 12 and into the priming chamber in readiness for the next discharging first stroke.

With such a pump the volume of solution discharged through the nozzle 26 is determined by the volume of the priming chamber. This volume is preferably chosen to be in the range 0.05ml to 0.15ml for the reasons previously mentioned and defines the volume unit dose (Vu) to be dispensed by the device 10. In the embodiment shown in Figure 1 the volume unit dose (Vu) is 0.1 ml.

The pump 20 is operably connected to the spray head 16 such that downward depression of the head 16 in the direction of arrows 'O' in Figure 1 causes the pump 20 to discharge the volume unit dose (Vu) of the solution in the form of spray 30.

Preferably, as with the dispenser disclosed in US patent 4565302, the dispenser 14 includes a counting means which is operated each time the pump 20 is operated to dispense a volume unit dose of solution 15. Preferably as indicated in the fifth embodiment of Figure 4, the counting means operates a visual display 34 which indicates to the operative (i.e. the patient or carer) the number of volume unit doses which have been administered from the device 10.

Other examples of electronic and dose counters for pharmaceutical dispensing devices which can be used with the device of the present invention are described in US patents 7347200, 7195134, 6769601, 6659307, 6651844 and 6446627.

Preferably as provided by the dispenser of US patent 4565302, the device 10 includes dispenser inactivation means which operates to prevent further discharge of the solution 15 after the predefined number (N) of volume unit doses have been dispensed. As indicated hereinbefore, the number (N) is preferably chosen to ensure that only full volume unit doses of solution are discharged from the device 10 in order to avoid the tail-off phenomenon. This number (N) needs to be chosen bearing in mind the fill volume (Vf) and also the residual volume (Vr) of the solution.

In this respect the minimum fill volume (Vf) of the solution contained in container 12 is given by the equation :

$$Vf = ( Vu \times N ) + P + Vr$$

Where Vu is the volume unit dose, N is the predefined number of volume unit doses to be administered by the device, P is the volume of solution required to initially prime the pump and fill the dip tube, and Vr is the residual amount of solution remaining in the container 12 after N full volume unit doses have been dispensed from the container 12.

It is recognised that the tail off phenomenon is caused by air entering the terminal end 23 of the dip tube when the container 12 nears to empty and so in accordance with the present invention Vr is chosen to be sufficiently great to maintain the terminal end 23 of the dip tube 22 submerged beneath the surface of the solution

15 after dispensing a volume unit dose for the Nth time.

Vr should be kept to a minimum in order to avoid unnecessary waste and preferably the volume (Vr) is less than 1ml, more preferably less than 0.8ml and most preferably less than 0.6ml.

In order to achieve volumes (Vr) of this order, the shape of the container 12 is adapted to reduce the volume (Vr) required to ensure that a full final Nth volume unit dose of solution is administered, particularly when bearing in mind that the container 12 is typically angled at about 30 degrees from the vertical when used.

This is illustrated for comparative purposes in Figure 2 wherein the container of Figure 2 A has a much wider internal dimension compared to the container of Figure 2B. It will be appreciated by comparing the containers of Figures 2A and 2B that the container of Figure 2A requires a much larger volume Vr to maintain the tip 23 of dip tube 22 submerged beneath the surface of the solution 15 (particularly when tipped at 30 degrees) than the container of Figure 2B.

Various designs of container 12 according to the present invention are illustrated by way of example in Figures 3A, 3B, and 3C; these containers share the common aim of providing a container having a relatively large external volume to provide stability for the container 12 when placed upon a surface, e.g. table top, whilst providing a reduced internal volume for retaining the solution 15 and thereby reduce volume Vr.

In Figure 3A, the reduced internal volume is achieved by increasing the thickness of the side walls 12a of the container 12.

In Figure 3B, the side walls 12a are also thickened to define an internal volume which is of inverted conical shape. This provides an internal bottom for the container 12a which is only slightly wider than the width of the dip tube 22 and so requires a small volume of solution to submerge the terminal end 23 of the tube 22.

In Figure 3C, the internal volume of the container 12 is defined by a narrow closed tube insert 12b. The internal diameter of the tube insert 12b is slightly greater than the external diameter of the dip tube 22 and so again requires only a small volume Vr to submerge the tip 23 of the dip tube 22.

These preferred shapes of container 12 may be expressed in terms of the 'volume ratio' of the internal volume to the external volume, i.e. if the container has an internal volume of 6ml and external volume of 10ml, then the container would have a volume ration of 0.6.

The internal volume of the container 12 is most conveniently measured by determining the weight of water required to fill the container to the brim and then, using the known density of water, calculating the volume.

The external volume of the container may be determined by measuring the external dimensions of the container and calculating the volume from the measured dimensions, or by measuring the weight of water displaced when the container is fully immersed in water and then using the known density of water to calculate the volume.

Preferably the volume ratio is in the range 0.15 to 0.9, more preferably 0.2 to 0.9, and most preferably 0.25 to 0.7.

The container 12 may be made from glass, plastics or a combination of both, for example a glass bottle with a plastics insert. The preferred plastic is a cyclic olefin copolymer.

The solution 15 contained within the container 12 is preferably an opioid solution which contains as an active component an opioid analgesic in a sufficient concentration to provide the desired therapeutic relief per volume unit dose (Vu).

Typically for a less potent opioid analgesic such as morphine, the amount by weight present in a volume unit dose (Vu) is in the range of 1 to 50 mg whereas for a more potent analgesic such as sufentanil the amount present by weight in a volume unit dose (Vu) is 1 to 200 micrograms.

An especially preferred opioid analgesic for use in the compositions of this invention is fentanyl. Fentanyl (N-(1-phenethyl-4-piperidyl)propionanilide) is a potent opioid analgesic agent used in the treatment of severe acute and chronic pain. By the term fentanyl we include its salts. Fentanyl is generally used in the form of the citrate salt.

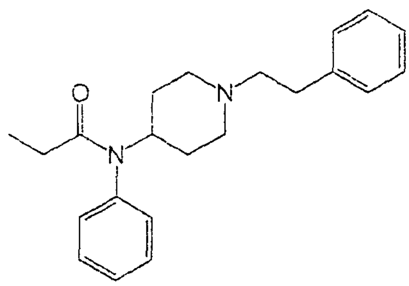

*Fentanyl*

The preferred intranasal dose of fentanyl, expressed as base, for treating a single episode of acute pain is in the range 10 to 2000 micrograms, more preferably 15 to 1500 micrograms, and most preferably 20 to 1000 micrograms. The preferred amount of fentanyl by weight, expressed as base, present in a volume unit dose (Vu) is in the range of 5 to 2000 micrograms.

*Example*

A preferred example of an intranasal device utilising fentanyl as the opioid analgesic is detailed below:-

(i) Drug solution: The solution is preferably predominantly aqueous and contains 0.1 to 20 mg/ml fentanyl, preferably in the form of the citrate salt, wherein 0.1 mg/ml and 20 mg/ml fentanyl are equivalent to approximately 0.16 mg/ml and 31.4 mg/ml fentanyl

citrate, respectively. The drug solution may optionally contain additives well known to one skilled in the art, such as preservatives, agents to adjust tonicity and acids or bases to adjust pH. Viscosity-modifying or gel-forming polymers, such as pectin, chitosan, celluloses or poloxamers may also be included. Examples of fentanyl intranasal compositions suitable for use in this invention may be found in WO 04/062561 and WO 02/09707.

(ii) Volume unit dose (Vu): The volume of each dose spray delivered by actuation of the spray pump is preferably 0.05 ml or 0.1 ml.

(iii) "Volume Ratio": The container preferably has a volume ratio, as defined earlier, in the range 0.3 to 0.7.

(iv) Fill volume (Vf): The bottle fill volume is preferably in the range 0.7 to 3.5 ml, more preferably in the range 0.8 to 3.0 ml, and most preferably in the range 0.9 to 2.5 ml, for example 1.0 to 1.8 ml.

(v) Number of volume unit doses (N): The number of dose sprays is preferably in the range 2 to 30, more preferably in the range 3 to 20, and most preferably in the range 3 to 16, for example 4 to 8.

The dispensing device 12 described above and illustrated in the accompanying drawings is particularly adapted for intranasal administration of the solution 15. It is however appreciated that the device may be adapted by adopting a suitably shaped nozzle instead of spray nozzle 26 to administer opioid analgesics by other routes, such as the oral cavity for buccal or sublingual absorption.

## Claims

**1.** A method of filling a device (10) for administering a predefined number (N) of predefined volume unit doses (Vu) of an opioid analgesic (15) in liquid form drawn from a container (12) containing a plurality of volume unit doses of the opioid analgesic (15) via a dip tube (22), the device (10) including:

a closed container (12) containing a predetermined fill volume (Vf) of the opioid analgesic (15);
a dispenser (14) connected to the container (12), the dispenser (14) being operable to administer an individual one volume unit dose (Vu) repeatedly said predefined number of times (N);
both the dispenser (14) being adapted and the concentration of said opioid analgesic (15) being chosen such that said volume unit dose is in the range 0.05 to 0.15ml, wherein the dispenser (14) includes counting means operable to count the number of one volume unit doses administered and further includes dispenser (14) deactivating means which is operated by the counting means to prevent further administration of one volume unit doses of the drug by the dispenser (14) when the counting means has counted said predefined number of times (N);
**characterised in that** the method comprises determining a fill volume (Vf) of the opioid analgesic (15) in accordance with the formula

$$Vf = (Vu \times N) + P + Vr$$

wherein P is the volume required for priming the pump that maintains a terminal end (23) of the dip tube (22) submerged beneath a surface of the liquid Vr is the residual volume of liquid opioid analgesic (15) remaining in the container (12) at the end of administering volume unit doses N times and which is sufficient to ensure N full volume unit doses are administered.

**2.** A method of filling a device (10) according to Claim 1 wherein the volume unit dose is 0.1 ml.

**3.** A method of filling a device (10) according to Claim 1 wherein the concentration of the opioid analgesic (15) in liquid form is in the range 0.005 mg/ml to 1000 mg/ml.

**4.** A method of filling a device (10) according to Claim 3 wherein the opioid analgesic (15) is a solution of fentanyl, the concentration of fentanyl in the solution being in the range of 0.1 to 20 mg/ml.

**5.** A method of filling a device (10) according to Claim 4 wherein the solution of fentanyl is a solution of fentanyl citrate, the concentration of fentanyl citrate in the solution being in the range of about 0.16 to 31.4 mg/ml.

**6.** A method of filling a device (10) according to any preceding claim wherein said predefined number (N) is in the range 2 to 30.

**7.** A method of filling a device (10) according to Claim 6 wherein the predefined number (N) is in the range 4 to 8.

**8.** A method of filling a device (10) according to Claim 6 or Claim 7 wherein the dispenser (14) is a nasal spray dispenser including a two-stroke pump which is operable on a complete first stroke in one direction to discharge said unit volume dose and operable on a complete second stroke in the opposite direction to replenish the pump, the pump further including a dip tube which extends into the container (12) in order to feed the liquid analgesic in the container (12) to the pump.

**9.** A method of filling a device (10) according to any preceding Claim wherein the container (12) has an internal/external volume ratio (as herein defined) in the range of 0.15 to 0.9.

**10.** A method of filling a device (10) according to claim 9 wherein the internal/external volume ratio is in the range of 0.25 to 0.7.

**11.** A method of filling a device (10) as claimed in any preceding Claim, wherein the deactivation means operates to prevent a tail off phenomenon being reached.

**12.** A method of filling a device (10) as claimed in any preceding Claim, wherein the container (12) is adapted to reduce the volume (Vr) required to ensure that a full final Nth volume unit dose is administered.

**13.** A method of filling a device (10) as claimed in Claim 12, wherein the container (12) has an internal volume which is of inverted conical shape.

**Patentansprüche**

**1.** Verfahren zum Befüllen einer Vorrichtung (10) zum Verabreichen einer vordefinierten Anzahl (N) von vordefinierten Volumeneinheitsdosen (Vu) eines Opioidanalgetikums (15) in flüssiger Form, die aus einem Behälter (12) entnommen werden, der mehrere Volumeneinheitsdosen des Opioidanalgetikums (15) enthält, über ein Tauchrohr (22), wobei die Vorrichtung (10) Folgendes einschließt:

einen geschlossenen Behälter (12), der ein vorbestimmtes Füllvolumen (Vf) des Opioidanalgetikums (15) enthält;
einen Spender (14), der mit dem Behälter (12) verbunden ist, wobei der Spender (14) betreibbar ist, um eine einzelne Einzelvolumeneinheitsdosis (Vu) die vordefinierte Anzahl von Malen (N) wiederholt zu verabreichen;
wobei sowohl der Spender (14) dazu ausgelegt ist, als auch die Konzentration des Opioidanalgetikums (15) so gewählt ist, dass die Volumeneinheitsdosis im Bereich von 0,05 bis 0,15 ml liegt, wobei der Spender (14) ein Zählmittel einschließt, das zum Zählen der Anzahl von Einzelvolumeneinheitsdosen, die verabreicht werden, betreibbar ist, und ferner ein Deaktivierungsmittel für den Spender (14) einschließt, das durch das Zählmittel betätigt wird, um eine weitere Verabreichung von Einzelvolumeneinheitsdosen des Arzneimittels durch den Spender (14) zu verhindern, wenn das Zählmittel die vordefinierte Anzahl von Malen (N) gezählt hat;
**dadurch gekennzeichnet, dass** das Verfahren das Bestimmen eines Füllvolumens (Vf) des Opioidanalgetikums (15) gemäß der nachstehenden Formel umfasst

$$Vf = (Vu \times N) + P + Vr$$

wobei P das Volumen ist, das für das Ansaugen der Pumpe erforderlich ist, die ein äußeres Ende (23) des Tauchrohrs (22) unter einer Oberfläche der Flüssigkeit eingetaucht hält, Vr das Restvolumen des flüssigen Opioidanalgetikums (15) ist, das in dem Behälter (12) am Ende der Verabreichung von Volumeneinheitsdosen N Mal verbleibt und das ausreichend ist, um sicherzustellen, dass N volle Volumeneinheitsdosen verabreicht werden.

**2.** Verfahren zum Befüllen einer Vorrichtung (10) nach Anspruch 1, wobei die Volumeneinheitsdosis 0,1 ml beträgt.

**3.** Verfahren zum Befüllen einer Vorrichtung (10) nach Anspruch 1, wobei die Konzentration des Opioidanalgetikums (15) in flüssiger Form im Bereich von 0,005 mg/ml bis 1000 mg/ml liegt.

**4.** Verfahren zum Befüllen einer Vorrichtung (10) nach Anspruch 3, wobei das Opioidanalgetikum (15) eine Fentanyllösung ist, wobei die Konzentration von Fentanyl in der Lösung im Bereich von 0,1 bis 20 mg/ml liegt.

**5.** Verfahren zum Befüllen einer Vorrichtung (10) nach Anspruch 4, wobei die Fentanyllösung eine Fentanylcitratlösung ist, wobei die Konzentration von Fentanylcitrat in der Lösung im Bereich von etwa 0,16 bis 31,4 mg/ml liegt.

**6.** Verfahren zum Befüllen einer Vorrichtung (10) nach einem vorhergehenden Anspruch, wobei die vordefinierte Zahl (N) im Bereich von 2 bis 30 liegt.

**7.** Verfahren zum Befüllen einer Vorrichtung (10) nach Anspruch 6, wobei die vordefinierte Zahl (N) im Bereich von 4 bis 8 liegt.

**8.** Verfahren zum Befüllen einer Vorrichtung (10) nach Anspruch 6 oder Anspruch 7, wobei der Spender (14) ein Nasensprayspender ist, der eine Doppelhubpumpe einschließt, die bei einem vollständigen ersten Hub in einer Richtung betrieben werden kann, um die Einheitsvolumendosis abzugeben und bei einem vollständigen zweiten Hub in der entgegengesetzten Richtung betrieben werden kann, um die

Pumpe wieder aufzufüllen, wobei die Pumpe ferner ein Tauchrohr einschließt, das sich in den Behälter (12) erstreckt, um das flüssige Analgetikum in dem Behälter (12) der Pumpe zuzuführen.

**9.** Verfahren zum Befüllen einer Vorrichtung (10) nach einem vorhergehenden Anspruch, wobei der Behälter (12) ein Innen-/Außenvolumenverhältnis (wie hierin definiert) im Bereich von 0,15 bis 0,9 aufweist.

**10.** Verfahren zum Befüllen einer Vorrichtung (10) nach Anspruch 9, wobei das Innen-/Außenvolumenverhältnis im Bereich von 0,25 bis 0,7 liegt.

**11.** Verfahren zum Befüllen einer Vorrichtung (10), wie in einem vorhergehenden Anspruch beansprucht, wobei das Deaktivierungsmittel betrieben wird, um zu verhindern, dass es zu Dosisschwankungen bei zunehmender Entleerung ["Tail-off-Phänomen"] kommt.

**12.** Verfahren zum Befüllen einer Vorrichtung (10), wie in einem vorhergehenden Anspruch beansprucht, wobei der Behälter (12) dazu ausgelegt ist, das Volumen (Vr) zu verringern, das erforderlich ist, um sicherzustellen, dass eine vollständige letzte N-te Volumeneinheitsdosis verabreicht wird.

**13.** Verfahren zum Befüllen einer Vorrichtung (10) nach Anspruch 12, wobei der Behälter (12) ein Innenvolumen aufweist, das eine umgekehrte konische Form aufweist.

## Revendications

**1.** Procédé de remplissage d'un dispositif (10) pour administrer un nombre prédéfini (N) de doses d'unité de volume prédéfinies (Vu) d'un analgésique opioïde (15) sous forme liquide prélevé à partir d'un récipient (12) contenant une pluralité de doses d'unité de volume de l'analgésique opioïde (15) via un tube plongeur (22), le dispositif (10) comprenant :

un récipient (12) fermé contenant un volume de remplissage préétabli (Vf) de l'analgésique opioïde (15) ;
un distributeur (14) relié au récipient (12), le distributeur (14) servant à administrer à un individu une dose d'unité de volume (Vu) de manière répétée ledit nombre prédéfini de fois (N) ;
à la fois le distributeur (14) étant adapté et la concentration dudit analgésique opioïde (15) étant choisie de sorte que ladite dose d'unité de volume se trouve dans la plage de 0,05 à 0,15 ml, ledit distributeur (14) comprenant un moyen de comptage servant à compter le nombre de doses d'une unité de volume administré et comprenant en outre un moyen de désactivation du distributeur (14) qui est actionné par le moyen de comptage pour empêcher l'administration supplémentaire de doses d'une unité de volume du médicament par le distributeur (14) lorsque le moyen de comptage a compté ledit nombre prédéfini de fois (N) ;
**caractérisé en ce que** le procédé comprend la détermination d'un volume de remplissage (Vf) de l'analgésique opioïde (15) conformément à la formule

$$Vf = (Vu \times N) + P + Vr$$

dans laquelle P représente le volume requis pour amorcer la pompe qui maintient une extrémité terminale (23) du tube plongeur (22) immergée sous la surface du liquide, Vr représente le volume résiduel d'analgésique opioïde liquide (15) restant dans le récipient (12) à la fin de l'administration de doses d'unité de volume N fois et qui est suffisant pour garantir que N doses d'unité de volume complètes soient administrées.

**2.** Procédé de remplissage d'un dispositif (10) selon la revendication 1, ladite dose d'unité de volume étant de 0,1 ml.

**3.** Procédé de remplissage d'un dispositif (10) selon la revendication 1, la concentration de l'analgésique opioïde (15) sous forme liquide se trouvant dans la plage de 0,005 mg/ml à 1000 mg/ml.

**4.** Procédé de remplissage d'un dispositif (10) selon la revendication 3, ledit analgésique opioïde (15) étant une solution de fentanyl, la concentration de fentanyl dans la solution se trouvant dans la plage de 0,1 à 20 mg/ml.

**5.** Procédé de remplissage d'un dispositif (10) selon la revendication 4, ladite solution de fentanyl étant une solution de citrate de fentanyl, la concentration de citrate de fentanyl dans la solution se trouvant dans la plage d'environ 0,16 à 31,4 mg/ml.

**6.** Procédé de remplissage d'un dispositif (10) selon l'une quelconque des revendications précédentes, ledit nombre prédéfini (N) se trouvant dans la plage de 2 à 30.

**7.** Procédé de remplissage d'un dispositif (10) selon la revendication 6, ledit nombre prédéfini (N) se trouvant dans la plage de 4 à 8.

**8.** Procédé de remplissage d'un dispositif (10) selon la revendication 6 ou la revendication 7, ledit distributeur (14) étant un distributeur par pulvérisation nasale comprenant une pompe à deux courses qui est utilisable sur une première course complète dans un sens pour décharger ladite dose de volume unitaire et utilisable sur une seconde course complète dans le sens opposé pour remplir la pompe, la pompe comprenant en outre un tube plongeur qui s'étend dans le récipient (12) afin d'alimenter l'analgésique liquide présent dans le récipient (12) vers la pompe.

**9.** Procédé de remplissage d'un dispositif (10) selon l'une quelconque des revendications précédentes, ledit récipient (12) présentant un rapport volume interne/volume externe (tel que défini dans la description) dans la plage de 0,15 à 0,9.

**10.** Procédé de remplissage d'un dispositif (10) selon la revendication 9, ledit rapport volume interne/volume externe se trouvant dans la plage de 0,25 à 0,7.

**11.** Procédé de remplissage d'un dispositif (10) selon l'une quelconque des revendications précédentes, ledit moyen de désactivation servant à empêcher qu'un phénomène de baisse ne soit atteint.

**12.** Procédé de remplissage d'un dispositif (10) selon l'une quelconque des revendications précédentes, ledit récipient (12) étant adapté à la réduction du volume (Vr) requis pour garantir qu'une Nième dose d'unité de volume finale complète soit administrée.

**13.** Procédé de remplissage d'un dispositif (10) selon la revendication 12, ledit récipient (12) ayant un volume interne qui est de forme conique inversée.

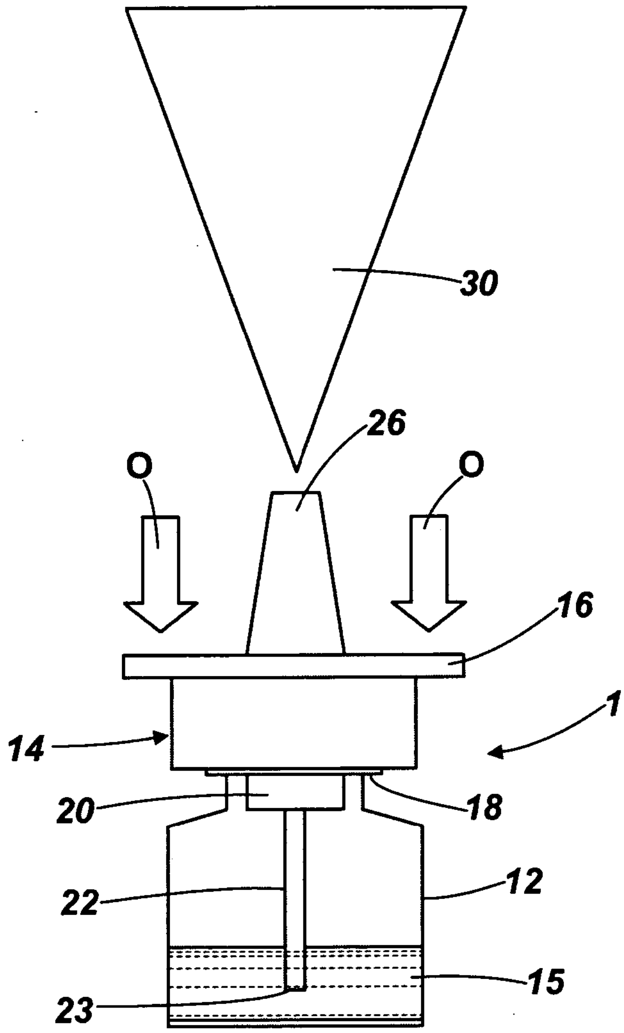


*Fig. 1*

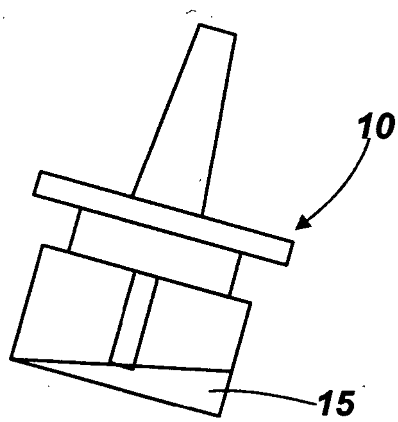


*Fig. 2A*

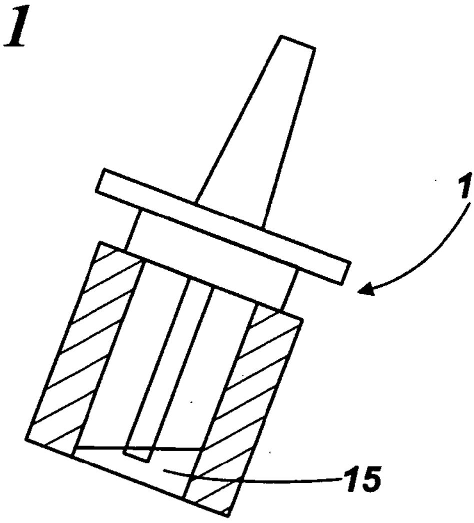


*Fig. 2B*

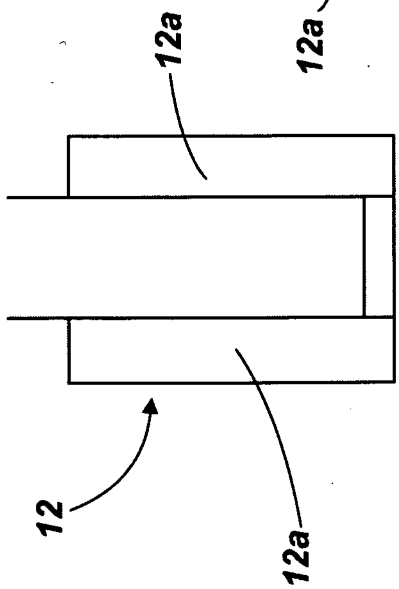


Fig. 3A

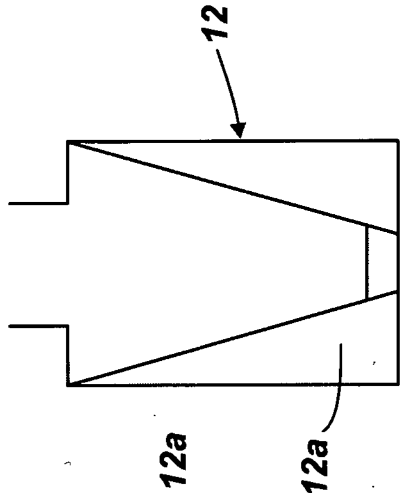


Fig. 3B

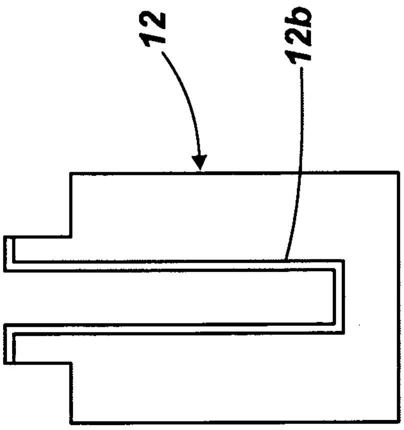


Fig. 3C

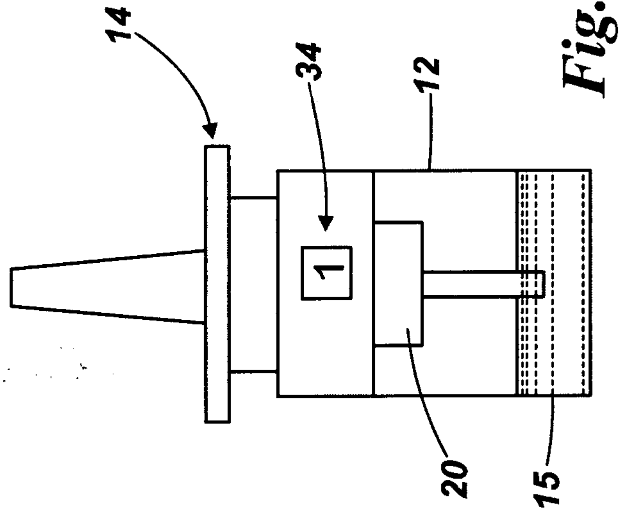


Fig. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4565302 A
- WO 0213886 A2
- US 2004135002 A1
- US 2007209660 A1
- US 6189739 B1
- US 5228586 A
- US 7347200 B
- US 7195134 B
- US 6769601 B
- US 6659307 B
- US 6651844 B
- US 6446627 B
- WO 04062561 A
- WO 0209707 A